# EUROPÄISCHE PATENTSCHRIFT

(11) **EP 1 680 150 B1**
(45) Veröffentlichungstag und Bekanntmachung des Hinweises auf die Patenterteilung: **04.11.2009**
(21) Anmeldenummer: 04797669.1
(22) Anmeldetag: 05.11.2004
(51) Int. Cl.: A61L 27/46, A61L 27/52, A61L 27/56

(54) **HYDROGELHALTIGES KOMPOSITMATERIAL, HERGESTELLT DURCH ELEKTRISCH-AUSGELÖSTE PRÄZIPITATION EINER FESTPHASE**
COMPOSITE MATERIAL CONTAINING HYDROGEN, PRODUCED BY THE ELECTRICALLY-TRIGGERED PRECIPITATION OF A SOLID PHASE
MATERIAU COMPOSITE CONTENANT DE L'HYDROGENE OBTENU PAR PRECIPITATION D'UNE PHASE SOLIDE DECLENCHEE ELECTRIQUEMENT

(30) Priorität: 05.11.2003 DE 10351661
(43) Veröffentlichungstag der Anmeldung: 19.07.2006
(73) Patentinhaber: Dritte Patentportfolio Beteiligungsgesellschaft mbH & Co. KG, 12529 Schönefeld / Waltersdorf (DE)
(72) Erfinder: ZEHBE, Rolf-Dieter, 13589 Berlin (DE); SCHUBERT, Helmut, 13465 Berlin (DE)
(74) Vertreter: Zech, Stefan Markus
(86) Internationale Anmeldenummer: PCT/EP2004/012564
(87) Internationale Veröffentlichungsnummer: WO 2005/044325

(56) Entgegenhaltungen:
- WO-A-03/022319
- WO-A-03/089022
- US-A- 3 892 649
- US-B1- 6 417 247
- KREKLAU B ET AL: "Tissue engineering of biphasic joint cartilage transplants" BIOMATERIALS, ELSEVIER SCIENCE PUBLISHERS BV., BARKING, GB, Bd. 20, Nr. 18, September 1999 (1999-09), Seiten 1743-1749, XP002228282 ISSN: 0142-9612
- MIKOS ANTONIOS G ET AL: "Formation of highly porous biodegradable scaffolds for tissue engineering" EJB ELECTRONIC JOURNAL OF BIOTECHNOLOGY, Bd. 3, Nr. 2 CITED SEPT. 6, 2000, 15. August 2000 (2000-08-15), Seiten 1-12 URL, XP001205451 ISSN: 0717-3458
- ZEHBE ET AL.: "Oriented Collagen-Based/Hydroxyapatite Matrices for Articular Cartilage Replacement" KEY ENGINEERING MATERIALS, Bd. 254-256, 7. November 2003 (2003-11-07), Seiten 1083-1086, XP009044597 SWITZERLAND

## Beschreibung

Die vorliegende Erfindung betrifft ein Verfahren zur Herstellung eines Kompositmaterials, ein dadurch hergestelltes Kompositmaterial und dessen Verwendung.

Im medizinischen Bereich werden zunehmend synthetische Gewebeträger als Ersatz für Organe und Gewebe benötigt. Dieses Gebiet wird allgemein als Tissue-Engineering bezeichnet. Von besonderer Bedeutung sind Gewebeträger, die mit dem nativen Organ oder Gewebe in den biologischen, biochemischen, biomechanischen und strukturellen Eigenschaften weitgehend übereinstimmen. Für die rekonstruktive und regenerative Medizin, die Traumatologie und die Orthopädie sind in den letzten Jahren Gewebeträger im muskuloskelettalen Bereich (Ersatz für Knochen, Knorpel und Sehnen) besonders bedeutsam geworden. Die Eigenschaften der zur Zeit verfügbaren Träger entsprechen jedoch in vielerlei Hinsicht nicht dem nativen Gewebe, da häufig die natürliche Morphologie nicht nachgebildet wird und/oder biochemische und/oder biomechanische Eigenschaften nicht übereinstimmen. Für die Reparatur von osteochondralen Defekten stellt Tissue-Engineering einen vielversprechenden Therapieansatz dar. Dabei werden Zellen mit einem Potential zur Knorpelbildung in poröse Trägermateralien eingebracht und dann direkt oder nach in vitro-Vorkultivierung in den chondralen Defekt in vivo eingesetzt. Diese Trägermaterialien müssen für ihren Einsatz spezifische Materialeigenschaften, insbesondere Formstabilität, verzögerten Abbau, Biokompatibilität, Zelladhärenz, Chondrokonduktivität, erfüllen. Von besonderer medizinischer und wirtschaftlicher Bedeutung ist das Gebiet der Gelenkserkranungen. Auf diesem medizinischen Gebiet sind die schmerzhaftesten Krankheiten diejenigen, bei denen Knorpel und die darunter liegende Knochenstruktur zerstört sind. Knorpel hat eine eingeschränkte Fähigkeit zur Regeneration und weist dann in der Regel eine geringere Qualität auf als der gesunde Knorpel. Zahlreiche Versuche sind unternommen worden, gesundes Knorpel- und subchondrales Knochengewebe zu transplantieren oder in Kultur zu halten; jedoch ist bislang auf diesem Wege kein erfolgreicher Ersatz geschaffen worden.

Um synthetische Materialien als erfolgreichen Knorpelersatz verwenden zu können, müssen viele unterschiedliche Eigenschaften des natürlichen Systems berücksichtigt werden. Zu diesen Eigenschaften zählen biochemische Zusammensetzung, strukturelle Identität (Nachahmung der zonalen Morphologie) und biomechanische Eigenschaften. Für diese Materialien sind poröse Schäume auf Grundlage von natürlichen und synthetischen Polymeren von unterschiedlichen Autoren vorgeschlagen worden. Ein guter Überblick über die Technologien zur Erzeugung von porösen Materialien für das Tissue-Engineerung wird von Mikos et al., Electronic Journal of Biotechnology, Vol. 3 No. 2, 2000 dargeboten. Die Autoren beschreiben mehrere Verfahren zur Erzeugung von hochporösen Gitterstrukturen. So werden bspw. Gitterstrukturen durch das Schaffen eines dreidimensionalen Netzwerks von Fasern aus Polyglycolsäure geschaffen. ("Fiber bonding"). Ein anderes Verfahren, um Poren in einer Matrix zu erzeugen, beinhaltet die Verwendung eines wasserlöslichen Porogens, wie etwa eines Salzes. Hierbei wird ein Polymer, Polymilchsäure oder Poly(DL-Milch-Co-Glycolsäure) in Chloroform oder Dichlormethan aufgelöst und dann in eine Petrischale gegossen, die mit dem Porogen gefüllt ist. Hierbei diffundiert das Porogen in die Polymermatrix ein. Nach Verdampfung des Lösungsmittels wird das Komposit aus Polymer/Porogen für 2 Tage in Wasser gelegt, um das Porogen zu entfernen. Die Porosität des resultierenden Gitters kann durch die Menge an zugegebenen Porogen kontrolliert werden, während die Porengröße von der Größe der Porogenteilchen, z.B., wenn das Porogen ein Salz ist, der Salzkristalle, abhängig ist. Ein weiteres Verfahren, das die Verwendung von organischen Lösungsmitteln bei der Porenbildung vermeidet, ist die Verwendung eines Gases als Porogen. Hierbei werden feste Platten eines Polymers formgepreßt und dann unter erhöhtem Druck einem Gas, bspw. CO₂ für einen längeren Zeitraum ausgesetzt. Dabei werden Porositäten von bis zu 93% und Porengrößen bis zu 100 µm erhalten, wobei die Poren jedoch größtenteils nicht miteinander verbunden sind. Zusätzliche Techniken, die für die Herstellung von porösen Polymergittem vorgeschlagen worden sind, beruhen auf dem Konzept der Phasentrennung anstelle des Einbaus eines Porogens. Solche Verfahren beinhalten die Emulgation/Gefriertrocknung oder eine Flüssig-Flüssigphasentrennung. Bei dem ersten Verfahren wird ein Polymer beispielsweise in Dichlormethan aufgelöst und dann wird destilliertes Wasser zugegeben, um eine Emulsion zu bilden. Die Polymer/Wassermischung wird in eine Form gegossen und abgeschreckt durch Einbringen in flüssigen Stickstoff. Nach Abschrecken werden die geschaffenen Gitterstrukturen bei -55°C gefriergetrocknet, was zur Entfernung des dispergierten Wassers und der Polymerlösungsmittel führt. Die Flüssig-Flüssigphasentrennung erzeugt Polymer-reiche und Polymer-arme Phasen innerhalb einer Polymerlösung. Die Polymer-arme Phase wird dann entfernt, was ein hochporöses Polymernetzwerk hinterläßt.

Eine Technik zur Phasentrennung eines Hydrogels wurde von Yannas und Mitarbeitern vorgeschlagen (US 4,955,893). Hierbei wird eine wäßrige Suspension von Collagen-Glycosaminoglycan innerhalb eines Röhrchens axial entlang des Röhrchens eingefroren, wodurch sich Eiskristalle ausbilden können. Anschließend wird das gefrorene Material unter Sublimationsbedingungen einem Vakuum ausgesetzt, wodurch die gebildeten Eiskristalle sublimierten und eine orientierte poröse Kanalstruktur hinterließen, die sich zur anschließenden Besiedelung mit Nervenzellen eignete. Eine ähnliche Technik wird in US 6,447,701 (Heschel et al.) beschrieben, wobei hier die Polymersuspension zwischen 2 Oberflächen mit unterschiedlichen Temperaturen gebracht wird, und wobei die Oberflächen einander gegenüberliegen und aufgrund der unterschiedlichen Temperatur eine im wesentlichen geordnete oder homogene Struktur des Polymemetzwerks und der sich darin ausbildenden Kristalle erzeugt wird.

WO 03/022319 beschreibt ein Verfahren zum Herstellen eines Gerüsts eines biokompatiblen Polymers, insbesondere eines Collagen-Hydrogels, welches mit einer Biokeramik vermischt und anschließend gefroren wird.

Keine der bislang im Stand der Technik beschriebenen Technologien hat zur Schaffung eines zufriedenstellenden Trägermaterials geführt, das die Anforderungen an biochemische Zusammesetzung, zonale Morphologie und biomechanische Eigenschaften gleichermaßen erfüllt.

Dementsprechend war es Aufgabe der vorliegenden Erfindung ein Verfahren bereitzustellen, mit dem sich Kompositmaterialien herstellen lassen, die zum Einsatz als Knochen-/Knorpelersatz geeignet sind, wobei die Kompositmaterialien sowohl eine naturnahe Gewebemorphologie aufweisen, biokompatibel sind, als auch in den biomechanischen und biochemischen Eigenschaften eine hervorragende Übereinstimmung mit nativem Gewebe zeigen.

Außerdem war es Aufgabe der vorliegenden Erfindung, ein entsprechendes Verfahren bereitzustellen, das einfach durchzuführen ist.

Weiterhin war es Aufgabe der vorliegenden Erfindung, ein Kompositmaterial bereitzustellen, das insbesondere die zonale Morphologie von natürlichem Gewebematerial, insbesondere Knorpel-/Knochengewebe in hervorragender Weise imitiert.

Diese Aufgaben werden gelöst durch ein Verfahren zur Herstellung eines Kompositmaterials, umfassend die folgenden Schritte:
a) Bereitstellen eines Hydrogels, das mindestens einen weiteren Bestandteil umfaßt, der bei Anlegen eines elektrischen Feldes an das Hydrogel ausfällt oder eine feste Phase bildet,
b) Anlegen eines elektrischen Feldes an das Hydrogel,
c) Induzieren einer Strukturierung, bevorzugt einer Porenbildung in dem Hydrogel.

In einer Ausführungsform werden die Schritte b) und c) zeitlich zusammen, voreinander oder nacheinander oder so durchgeführt, daß einer der beiden Schritte nach Beginn des jeweiligen anderen Schrittes angefangen wird, ohne daß der andere Schritt bereits abgeschlossen ist.

Bevorzugt wird Schritt c) durch einen Einfrierprozess des Hydrogels und/oder durch Gefriertrocknung des Hydrogels und/oder durch Elektrolyse von Wasser und/oder durch Elektrolyse von wässrigen Lösungen in dem Hydrogel durchgeführt.

In einer Ausführungsform erfolgt Schritt b) mittels mindestens zweier gegensätzlich gepolter Elektroden.

In einer Ausführungsform bildet in Schritt b) der mindestens eine weitere Bestandteil, der bei Anlegen eines elektrischen Feldes an das Hydrogel ausfällt oder eine feste Phase bildet, eine kristalline und/oder amorphe Phase oder eine Kombination kristalliner und amorpher Phasen.

Bevorzugt wird in Schritt b) eine Spannung von 3 V bis 20 V an das Hydrogel angelegt, und/oder ein Strom der Stärke von 0,5 A bis 5 A fließt durch das Hydrogel, bevorzugt über einen Zeitraum von 0,5 Minuten bis 120 Minuten.

Die Spannung ist eine Gleichspannung oder eine Wechselspannung. In einer Ausführungsform hat die Wechselspannung eine Frequenz im Bereich von 1 Hertz bis 0,01 Hertz.

In einer Ausführungsform ist das Hydrogel ein Hydrogel von einer oder mehreren Verbindungen, ausgewählt aus der Gruppe, umfassend Kollagen, insbesondere Kollagen vom Typ I und II, Telopeptid-freies Kollagen, Kollagenhydrolysate, Proteoglycane, Glycosaminoglycane, Polymethacrylsäuren, Polymethacrylate, Polyvinylpyrrolidon, Polyvinylalkohol, Gelatine, Polyglycolsäure, Polymilchsäure und Copolymere von Polymilchsäure und Polyglycolsäure, Glucose, Lipide, Phospholipide, Urate, Hyaluronsäure, Hyaluronsäurederivate, insbesondere Hyaluronsäureester, sowie ionische Bestandteile ausgewählt aus der Gruppe, umfassend Na⁺, K⁺, Mg²⁺, Ca²⁺, Cl⁻, HCO₃⁻, HPO₄²⁻, SO₄²⁻, F⁻.

In einer Ausführungsform ist der mindestens eine weitere Bestandteil, der bei Anlegen eines elektrischen Feldes an das Hydrogel ausfällt oder eine feste Phase bildet, ausgewählt aus der Gruppe, umfassend Calciumcarbonate, Calciumphosphate, insbesondere Hydroxylapatit, Tri-Calciumphosphate, Brushit, Octacalciumphosphat, amorphes Calciumphosphat, Tetracalciumphosphat, Monetit, calciumdefizitäres Hydroxylapatit, sowie Verbindungen, gebildet aus ionischen Bestandteilen, ausgewählt aus der Gruppe, umfassend Na⁺, K⁺, Mg²⁺, Ca²⁺, Cl⁻, HCO₃⁻, HPO₄²⁻, SO₄²⁻, F⁻ sowie ionische Bestandteile.

In einer Ausführungsform umfaßt das Hydrogel einen Bestandteil, der elektrisch leitfähig ist, wobei dieser Bestandteil mit dem mindestens einem weiteren Bestandteil, der bei Anlegen eines elektrischen Feldes an das Hydrogel ausfällt oder eine feste Phase bildet, identisch ist oder von diesem verschieden ist.

Bevorzugt wird der elektrisch leitfähige Bestandteil in das Hydrogel eingebracht oder auf das Hydrogel aufgetragen.

In einer Ausführungsform ist der elektrisch leitfähige Bestandteil chemisch und/oder biologisch inert, wobei bevorzugt ist, daß der elektrisch leitfähige Bestandteil ausgewählt ist aus der Gruppe, umfassend Edelmetalle, insbesondere elementares Gold und/oder Platin, sowie Kohlenstoff, insbesondere Graphit.

Sofern der elektrisch leitfähige Bestandteil in das Hydrogel eingebracht wird, ist bevorzugt, daß er dort homogen oder inhomogen im Hydrogel verteilt wird.

Sofern der elektrisch leitfähige Bestandteil auf einer Oberfläche des Hydrogels aufgetragen wird, ist bevorzugt, daß er durch eine Oberflächenbehandlung strukturiert wird.

In einer Ausführungsform liegt das Hydrogel als eine Schicht vor, die vor oder nach Durchführung des Schrittes b) und/oder c) aufgerollt wird.

Bevorzugt wird das Hydrogel chemisch und/oder physikalisch quervernetzt.

In einer Ausführungsform erfolgt die Gefriertrocknung durch Einfrieren des Hydrogels auf eine Temperatur im Bereich von -1°C bis -196°C, bevorzugt im Bereich von -10°C bis -50°C und anschließende Sublimation.

In einer Ausführungsform erfolgt das Einfrieren des Hydrogels gerichtet und/oder ungerichtet, bevorzugt von einer und/oder mehren Seiten des Hydrogels her, wobei bevorzugt wird, daß das Einfrieren des Hydrogels über einen Zeitraum von näherungsweise einer halben Stunde bis vier Stunden erfolgt. Ein Beispiel für ein ungerichtetes Einfrieren ist eine sehr schnelle Erstarrung (Schockgefriervorgang), die zu einem amorphen, glasartigen Zustand des Hydrogels führt.

Die Aufgaben der Erfindung werden auch gelöst durch ein Kompositmaterial, hergestellt durch ein Verfahren gemäß der vorliegenden Erfindung.

In einer Ausführungsform hat das Kompositmaterial der vorliegenden Erfindung eine porenaufweisende Schicht eines Gels, bevorzugt eines partiell hydratisierten Hydrogels oder eines Xerogels, an die eine feste Phase, bevorzugt eine kristalline und/oder amorphe Phase oder eine Kombination kristalliner und/oder amorpher Phasen angehängt ist, wobei bevorzugt ist, daß die Porengröße im Bereich von 10 µm - 150 µm liegt.

Wie hierin verwendet, ist unter " Porengröße" der mittlere Porenkanaldurchmesser zu verstehen.

In einer Ausführungsform ist die feste, bevorzugt kristalline und/oder amorphe Phase oder die Kombination kristalliner und amorpher Phasen ein Calciumphosphat

In einer Ausführungsform umfaßt das Kompositmaterial der vorliegenden Erfindung weiterhin mindestens eine das Zellwachstum oder die Zellansiedlung oder die Zelladhäsion fördernde Substanz, die bevorzugt ein Wachstumsfaktor oder ein fetales Serum oder Poly-L-Lysin ist. In einer Ausführungsform umfaßt das Kompositmaterial Serum, wobei das Serum autogenen, syngenen, allogenen oder xenogenen Ursprungs ist.

In einer bevorzugten Ausführungsform ist die mindestens eine das Zellwachstum oder die Zellansiedlung oder die Zelladhäsion fördernde Substanz Serum, wobei das Serum autogenen, syngenen, allogenen, oder xenogenen Ursprungs ist. Bevorzugter ist der Wachstumsfaktor ausgewählt aus der Gruppe, umfassend Substanzen der TGF-β-Superfamilie, insbesondere TGF-β1. In einer Ausführungsform ist das fetale Serum ein tierisches fetales Serum, beispielsweise fetales Kälberserum.

In einer Ausführungsform umfaßt das Kompositmaterial weiterhin biologische Zellen, bevorzugt menschliche oder tierische Zellen.

In einer anderen Ausführungsform umfaßt das Kompositmaterial pflanzliche Zellen.

Die Aufgaben der Erfindung werden auch gelöst durch die Verwendung eines Kompositmaterials gemäß der vorliegenden Erfindung als Träger für biologische Zellen, bevorzugt menschliche oder tierische oder pflanzliche Zellen.

Weiterhin werden die Aufgaben der vorliegenden Erfindung gelöst durch ein Kompositmaterial gemäß der vorliegenden Erfindung zur Verwendung als Gewebeersatz im menschlichen oder tierischen Körper.

Weiterhin werden die Aufgaben der Erfindung gelöst durch die Verwendung eines Kompositmaterials gemäß der vorliegenden Erfindung als Trägermaterial für biologisch und/oder chemisch und/oder katalytisch wirksame Substanzen in den Bereichen der Abwasserreinigung, Filtration, Bioreaktortechnik und/oder Katalyse.

Wie hierin verwendet bezeichnet der Begriff "Hydrogel" ein wasserenthaltendes Gel auf der Basis hydrophiler Moleküle, bevorzugt Polymere, die als dreidimensionale Netzwerke vorliegen. In Wasser quellen diese Netzwerke unter weitgehender Formerhaltung bis zu einem Gleichgewichtsvolumen auf. Die Netzwerkbildung erfolgt vorwiegend über chemische Verknüpfung der einzelnen Polymerketten, ist aber auch physikalisch durch elektrostatische, hydrophobe oder Dipol/Dipol-Wechselwirkungen zwischen einzelnen Segmenten der Molekülketten möglich.

Unter "Xerogelen", wie hierin verwendet, sind Gele zu verstehen, die ihre Flüssigkeit auf irgendeine Weise, bspw. durch Verdampfen, Abpressen oder Absaugen, verloren haben, wobei sich auch die räumliche Anordnung des Netzes verändert haben kann, so daß die Abstände zwischen den Strukturelementen andere Dimensionen als in dem Hydrogel haben.

Unter "Gelen" allgemein, wie hierin verwendet, sind formbeständige, leicht deformierbare, an Flüssigkeiten und/oder Gasen reiche disperse Systeme aus mindestens zwei Komponenten zu verstehen, die zumeist aus einem festen, kolloid zerteilten Stoff mit langen oder stark verzweigten Teilchen und einer Flüssigkeit (meist Wasser) als Dispersionsmittel bestehen. In der Regel ist die feste Substanz kohärent, das heißt sie bildet im Dispersionsmittel ein räumliches Netzwerk, wobei die Teilchen durch Neben- oder Hauptvalenzen an verschiedenen Punkten aneinander haften.

Das Anlegen eines elektrischen Feldes an das erfindungsgemäße Hydrogel kann über Elektroden an sich bekannter Bauweise erfolgen. In einer bevorzugten Ausführungsform werden 2 Elektroden verwendet, die einander an gegenüberliegenden Seiten des Hydrogels mit diesem in elektrisch leitender Verbindung in Kontakt treten. Das Verfahren der Gefriertrocknung des Hydrogels ist an sich bekannt und im Stand der Technik beschrieben (e. g. US 4,955,893). Bevorzugt erfolgt der Einfrierschritt des Hydrogels in einer gerichteten Weise, so daß die Ausbildung von Eiskristallen zellular und/oder dendritisch strukturiert erfolgt und/oder auf eine Art erfolgt, die durch die physikalischen Gesetze bei der Erstarrung wasserhaltiger Substanzen vorgegeben ist. Der Einfrierschritt kann jedoch auch ungerichtet erfolgen. Ein Beispiel für ein ungerichtetes Einfrieren ist eine sehr schnelle Erstarrung (Schockgefriervorgang), beispielsweise über einen Zeitraum von 1 s - 180 s, die zu einem amorphen glasartigen Zustand des Hydrogels führt.

In einer bevorzugten Ausführungsform wird das Hydrogel mit der sich ausbildenden festen Phase des mindestens einen weiteren Bestandteils kovalent verbunden. Dies kann bspw. über chemische Quervernetzung oder physikalische Quervernetzung erfolgen. Die physikalische Quervernetzung kann durch Bestrahlung (meist kurzwellige Strahlung, wie UV, Gamma, Röntgen) und/oder thermische Behandlung erfolgen. Die Wirkungsweise ist prinzipiell ähnlich wie bei der chemischen Quervernetzung (reaktive chemische Gruppen oder Moleküle die mit anderen Molekülen oder Gruppen chemische Bindungen bilden und so zu einer Stabilisierung der Struktur führen).

In einer bevorzugten Ausführungsform ist der vom Hydrogel umfaßte elektrisch leitfähige Bestandteil biokompatibel.

Es wird nunmehr Bezug genommen auf die Abbildungen, bei denen
Abbildung 1 einen Aufbau zur Erzeugung einer Ausführungsform eines Kompositmaterials gemäß der vorliegenden Erfindung darstellt,
Abbildung 2A eine Ausführungsform eines gemäß dem erfindungsgemäßen Verfahren hergestellten Kompositmaterials zeigt,

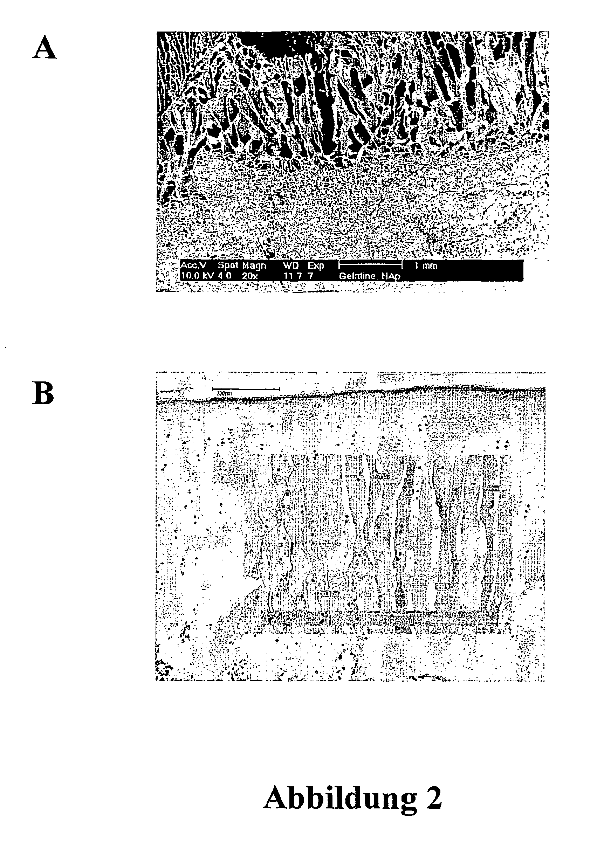

Abbildung 2B eine andere Ausführungsform eines gemäß dem erfindungsgemäßen Verfahren hergestellten Kompositmaterials zeigt, welches mit einem zellhistologischen Bild von Knorpel, gefärbt mit Hematoxylin-Eosin, überlagert ist. Der in Abbildung 2B eingesetzte kleinere Rahmen zeigt das Kompositmaterial gemäß der vorliegenden Erfindung, während der größere Rahmen der Abbildung 2B natürlichen Knorpel zeigt. Zellkörper in dem Knorpel sind als dunkle Körperchen , angefärbt mit Hematoxylin-Eosin, zu erkennen. Die Überlagerung der Bilder zeigt die gute Übereinstimmung des erfindungsgemäßen Kompositmaterials mit der natürlichen Struktur gesunden Knorpels.

Die Erfindung wird nunmehr anhand der folgenden Beispiele beschrieben, die zu Erläuterungszwecken dargeboten werden, nicht um die Erfindung zu beschränken.

### Beispiel 1

Der Versuchsaufbau, bestehend aus Peltier-Element und zugehörigem Kühlkörper, sowie zwei Platin-Elektroden und dem Mantel des zylindrischen Probengefäßes, wird steril vorbereitet. Die beiden Platin-Elektroden, von denen eine Elektrode den Boden des Probengefäßes bildet, werden zusammen mit einem PTFE-Rohr, welches den Mantel des zylindrischen Probengefäßes bildet, in einem Druckautoklaven unter Sattdampfatmosphäre bei 121°C und max. 3 bar Druck für 30 min sterilisiert. Andere in der Medizin übliche Sterilisationsprozesse sind ebenfalls möglich.

Das Peltier-Element, welches als Kühl- und Heizkörper dient, wird durch geeignete Polung an der Oberfläche, die Kontakt zum Probengefäß hat, erwärmt auf 40°C. 5 g Gelatine (Sigma, Typ A aus Schweinehaut) werden bei 50°C in 40 ml einer gesättigten Calciumphosphat Lösung homogen gelöst. 5 ml der Gelatine-Lösung werden anschließend in das sterile Probengefäß der Versuchsapparatur eingefüllt. Nach dem Einfüllen der Gelatine-Lösung wird die obere Platin-Elektrode in die Lösung getaucht.

Anschließend werden folgende Schritte durchgeführt:
1a) Durch Umpolung des Peltier-Elements wird eine Temperatur von -30°C an der Oberfläche, die Kontakt zum Probengefäß hat, eingestellt. Gleichzeitig wird eine Spannung von 15 V an den Platin-Elektroden eingestellt, wodurch es zur elektrolytischen Zersetzung des Wassers kommt und Gasblasen aus H₂ und O₂ entstehen. Außerdem kommt es zur Bildung einer Calciumphosphatschicht an der unteren Elektrode. Sobald eine geschlossene Eisschicht an der Elektrode entsteht, die den Boden des Probengefäßes bildet, hört die Gasentwicklung auf, da kein Stromfluss mehr möglich ist.
1b) Anders als in 1a) findet die elektrolytische Zersetzung des Wassers bereits vor Umpolung des Peltier-Elements (und der damit verbundenen Temperaturerniedrigung auf -30°C) statt und endet erst wieder, wenn sich eine geschlossene Eisschicht gebildet hat.
   Nach den alternativen Schritten 1a) oder 1b) ist die Lösung nach ca. 2h vollständig gerichtet erstarrt und wird mit einem sterilen Stempel aus dem Mantel des Probengefäßes gedrückt und sofort in einen Gefriertrockner transferiert. Nach 12h Gefriertrocknung wird das getrocknete Hydrogel dem Gefriertrockner entnommen.
1c) Das getrocknete Hydrogel wird chemisch in einer aldehyd-haltigen Atmosphäre für 6h vernetzt. Anschließend werden drei Waschungen in steriler 0,9 %iger Kochsalzlösung durchgeführt, um Aldehydreste im getrockneten Hydrogel zu eliminieren.
1d) Das getrocknete Hydrogel wird thermisch in trockener Atmosphäre bei 120°C vernetzt.

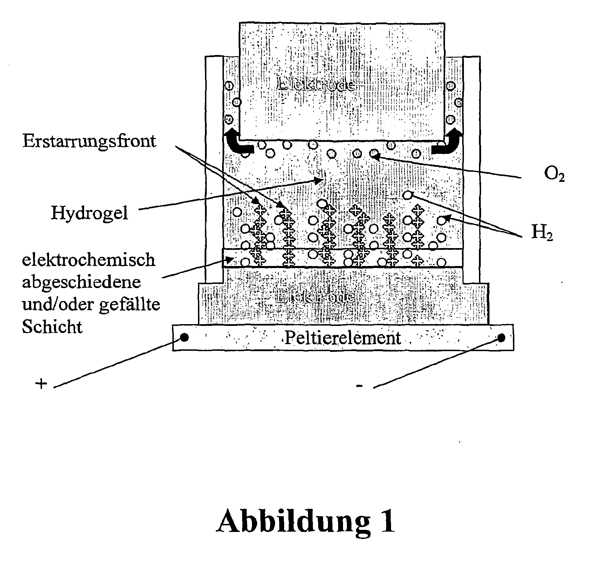

Ein im Rahmen dieses Beispiels hergestelltes Kompositmaterial kann in den Abbildungen 1, 2A und 2B (kleiner Rahmen) betrachtet werden.

### Beispiel 2

Eigenschaften des erfindungsgemäßen Kompositmaterials aus Beispiel 1.
2a) Die mechanischen Eigenschaften des erfindungsgemäßen Kompositmaterials wurden mit einem speziellen Prüfaufbau getestet.
Die Materialeigenschaften wurden wie folgt ermittelt:

| Literatur | Bemerkung | E-Modul [MPa] | Poisson Zahl |
|---|---|---|---|
| Korhonen et al., J. Biomech., 903-909, 2002 | (Prüf-Stempel-Ø = 3.7mm) bovine Explantate | | |
| | Femur | 0.47 ± 0.15 | 0.26 ± 0.08 |
| | Humerus | 1.15 ± 0.43 | 0.16 ± 0.06 |
| | Patella | 0.72 ± 0.19 | 0.21 ± 0.05 |
| erfindungs-gemäßes Komposit-material | (Prüf-Stempel-Ø = 8.0mm) erfindungsgemäßes Kompositmaterial | 0.67 ± 0.02 | 0.27 |

Unter E-Modul ist das Elastizitätsmodul, eine materialspezifische Kenngröße zu verstehen. Es stellt den Proportionalitätsfaktor in dem als Hookschem Gesetz bekannten Zusammenhang dar. Unter der Poisson-Zahl ist die Querkontraktion zu verstehen, sie beschreibt das Verhältnis zwischen Querschnittsänderung und Längenänderung eines Materials.
Die mechanische Prüfung wurde in 0,9%iger Kochsalzlösung durchgeführt. Die mechanischen Eigenschaften des erfindungsgemäßen Kompositmaterials liegen angenähert im Bereich von natürlichem Gewebe.
2b) Die Durchmesser der im erfindungsgemäßen Kompositmaterial gebildeten Porenkanäle wurden durch Auswertung rasterelektronenmikroskopischer Bilder ermittelt. Der einstellbare Porenkanaldurchmesser liegt im Bereich zwischen 10 µm und 150 µm und begünstigt eine einfache Besiedlung der Porenkanäle durch Zellen sowie deren gute Nährstoffversorgung mittels Diffusion. Bei der Zellbesiedlung wird eine Zellsuspension in das erfindungsgemäße Kompositmaterial eingebracht, und die dermaßen angesiedelten Zellen bilden später das sehr dichte natürliche Fasergeflecht, wie es im gesunden Knorpel gefunden wird. Somit ermöglicht das erfindungsgemäße Kompositmaterial die Bildung von natürlichem Knorpel-/Knochengewebe in hervorragender Weise, weist nach Besiedelung eine ähnliche Gewebemorphologie auf, zeigt in den biomechanischen Eigenschaften eine hervorragende Übereinstimmung mit nativem Gewebe und ist biokompatibel.

## Patentansprüche

1. Verfahren zur Herstellung eines zum Einsatz als Knochen-/Knorpelersatz geeigneten Kompositmaterials, umfassend die folgenden Schritte:
a) Bereitstellen eines Hydrogels, das mindestens einen weiteren Bestandteil umfaßt, der bei Anlegen eines elektrischen Feldes an das Hydrogel ausfällt oder eine feste Phase bildet,
b) Anlegen eines elektrischen Feldes an das Hydrogel,
c) Induzieren einer Strukturierung, bevorzugt einer Porenbildung in dem Hydrogel.

2. Verfahren nach Anspruch 1, wobei die Schritte b) und c) zeitlich zusammen, voreinander oder nacheinander oder so durchgeführt werden, daß einer der beiden Schritte nach Beginn des jeweiligen anderen Schrittes angefangen wird, ohne daß der andere Schritt bereits abgeschlossen ist.

3. Verfahren nach einem der vorangehenden Ansprüche, **dadurch gekennzeichnet, daß** Schritt c) durch einen Einfrierprozess und/oder durch Gefriertrocknung des Hydrogels und/oder durch Elektrolyse von Wasser und/oder durch Elektrolyse von wässrigen Lösungen in dem Hydrogel durchgeführt wird.

4. Verfahren nach einem der vorangehenden Ansprüche, **dadurch gekennzeichnet, daß** Schritt b) mittels mindestens zweier gegensätzlich gepolter Elektroden erfolgt.

5. Verfahren nach einem der vorangehenden Ansprüche, **dadurch gekennzeichnet, daß** in Schritt b) der mindestens eine weitere Bestandteil, der bei Anlegen eines elektrischen Feldes an das Hydrogel ausfällt oder eine feste Phase bildet, eine kristalline und/oder amorphe Phase oder eine Kombination kristalliner und amorpher Phasen bildet.

6. Verfahren nach einem der vorangehenden Ansprüche, **dadurch gekennzeichnet, daß** in Schritt b) eine Spannung von 3 V bis 20 V an das Hydrogel angelegt und/oder ein Strom der Stärke von 0,5 A bis 5 A durch das Hydrogel fließt, bevorzugt über einen Zeitraum von 0,5 Minuten bis 120 Minuten.

7. Verfahren nach Anspruch 6, **dadurch gekennzeichnet, daß** die angelegte Spannung eine Gleichspannung oder eine Wechselspannung ist.

8. Verfahren nach einem der vorangehenden Ansprüche, **dadurch gekennzeichnet, daß** das Hydrogel ein Hydrogel von einer oder mehreren Verbindungen ist, ausgewählt aus der Gruppe, umfassend Kollagen, insbesondere Kollagen vom Typ I und II, Telopeptid-freies Kollagen, Kollagenhydrolysate, Proteoglycane, Glycosaminoglycane, Polymethacrylsäuren, Polymethacrylate, Polyvinylpyrrolidon, Polyvinylalkohol, Gelatine, Polyglycolsäure, Polymilchsäure, Copolymere von Polymilchsäure und Polyglycolsäure, Glucose, Lipide, Phospholipide, Urate, Hyaluronsäure, Hyaluronsäurederivate, insbesondere Hyaluronsäureester, sowie ionische Bestandteile, ausgewählt aus der Gruppe, umfassend Na⁺, K⁺, Mg²⁺, Ca²⁺, Cl⁻, HCO₃⁻, HPO₄²⁻, SO₄²⁻, F⁻.

9. Verfahren nach einem der vorangehenden Ansprüche, **dadurch gekennzeichnet, daß** der mindestens eine weitere Bestandteil, der bei Anlegen eines elektrischen Feldes an das Hydrogel ausfällt oder eine feste Phase bildet, ausgewählt ist aus der Gruppe, umfassend Calciumcarbonate, Calciumphosphate, insbesondere Hydroxylapatit, Tri-Calciumphosphate, Brushit, Octacalciumphosphat, amorphes Calciumphosphat, Tetracalciumphosphat, Monetit, calciumdefizitätres Hydroxylapatit, sowie Verbindungen gebildet aus ionischen Bestandteilen, ausgewählt aus der Gruppe, umfassend Na⁺, K⁺, Mg²⁺, Ca²⁺, Cl⁻, HCO₃⁻, HPO₄²⁻, SO₄²⁻, F⁻.

10. Verfahren nach einem der vorangehenden Ansprüche, **dadurch gekennzeichnet, daß** das Hydrogel einen Bestandteil umfaßt, der elektrisch leitfähig ist, wobei dieser Bestandteil mit dem mindestens einem weiteren Bestandteil, der bei Anlegen eines elektrischen Feldes an das Hydrogel ausfällt oder eine feste Phase bildet, identisch ist oder von diesem verschieden ist.

11. Verfahren nach Anspruch 10, **dadurch gekennzeichnet, daß** der elektrisch leitfähige Bestandteil in das Hydrogel eingebracht wird oder auf das Hydrogel aufgetragen wird.

12. Verfahren nach einem der Ansprüche 10 - 11, **dadurch gekennzeichnet, daß** der elektrisch leitfähige Bestandteil chemisch und/oder biologisch inert ist.

13. Verfahren nach Anspruch 12, **dadurch gekennzeichnet, daß** der elektrisch leitfähige Bestandteil ausgewählt ist aus der Gruppe, umfassend Edelmetalle, insbesondere elementares Gold und/oder Platin, sowie Kohlenstoff, insbesondere Graphit.

14. Verfahren nach einem der Ansprüche 11 - 13, **dadurch gekennzeichnet, daß**, sofern der elektrisch leitfähige Bestandteil in das Hydrogel eingebracht wird, er dort homogen oder inhomogen im Hydrogel verteilt wird.

15. Verfahren nach einem der Ansprüche 11 - 13, **dadurch gekennzeichnet, daß**, sofern der elektrisch leitfähige Bestandteil auf einer Oberfläche des Hydrogels aufgetragen wird, er durch eine Oberflächenbehandlung strukturiert wird.

16. Verfahren nach einem der vorangehenden Ansprüche, **dadurch gekennzeichnet, daß** das Hydrogel als eine Schicht vorliegt, die vor oder nach Durchführung des Schrittes b) und/oder c) aufgerollt wird.

17. Verfahren nach einem der vorangehenden Ansprüche, **dadurch gekennzeichnet, daß** das Hydrogel chemisch und/oder physikalisch quervernetzt wird.

18. Verfahren nach einem der Ansprüche 3 - 17, **dadurch gekennzeichnet, daß** die Gefriertrocknung durch Einfrieren des Hydrogels auf eine Temperatur im Bereich von -1°C bis -196°C und anschließende Sublimation erfolgt.

19. Verfahren nach Anspruch 18, **dadurch gekennzeichnet, daß** das Einfrieren des Hydrogels gerichtet und/oder ungerichtet, bevorzugt von einer und/oder mehreren Seiten des Hydrogels her erfolgt.

20. Verfahren nach einem der Ansprüche 18 - 19, **dadurch gekennzeichnet, daß** das Einfrieren des Hydrogels über einen Zeitraum von näherungsweise einer halben Stunde bis vier Stunden erfolgt.

21. Kompositmaterial hergestellt durch ein Verfahren nach einem der vorangehenden Ansprüche.

22. Kompositmaterial nach Anspruch 21, **gekennzeichnet durch** eine porenaufweisende Schicht eines Gels, bevorzugt eines partiell hydratisierten Hydrogels oder eines Xerogels, an die eine feste Phase, bevorzugt eine kristalline und/oder amorphe Phase oder eine Kombination kristalliner und amorpher Phasen angehängt ist.

23. Kompositmaterial nach einem der Ansprüche 21 - 22, **gekennzeichnet durch** eine Porengröße von 10 µm - 150 µm.

24. Kompositmaterial nach einem der Ansprüche 21 - 23, **dadurch gekennzeichnet, daß** die feste, bevorzugt kristalline und/oder amorphe Phase oder die Kombination kristalliner und amorpher Phasen ein Calciumphosphat ist.

25. Kompositmaterial nach einem der Ansprüche 21 - 24, weiterhin umfassend mindestens eine das Zellwachstum oder die Zellansiedlung oder die Zelladhäsion fördernde Substanz.

26. Kompositmaterial nach Anspruch 25, **dadurch gekennzeichnet, daß** die mindestens eine das Zellwachstum oder die Zellansiedlung oder die Zelladhäsion fördernde Substanz ein Wachstumsfaktor oder ein fetales Serum oder Poly-L-Lysin ist, wobei der Wachstumsfaktor bevorzugt ausgewählt ist aus der Gruppe, umfassend Substanzen der TGF-β-Superfamilie, insbesondere TGF-β1, und wobei das fetale Serum ein tierisches fetales Serum, beispielsweise fetales Kälberserum ist.

27. Kompositmaterial nach Anspruch 25, **dadurch gekennzeichnet, daß** die mindestens eine das Zellwachstum oder die Zellansiedlung oder die Zelladhäsion fördernde Substanz Serum ist, wobei das Serum autogenen, syngenen, allogenen oder xenogenen Ursprungs ist.

28. Kompositmaterial nach einem der Ansprüche 21 - 27, **dadurch gekennzeichnet, daß** es weiterhin biologische Zellen, bevorzugt menschliche oder tierische Zellen oder pflanzliche Zellen umfaßt.

29. Verwendung eines Kompositmaterials nach einem der Ansprüche 21 - 28 als Träger für biologische Zellen, bevorzugt menschliche oder tierische Zellen oder pflanzliche Zellen.

30. Kompositmaterial nach einem der Ansprüche 21 - 28, zur Verwendung als Gewebeersatz im menschlichen oder tierischen Körper.

## Claims

1. A method of manufacturing a composite material suitable for use as replacement bone/cartilage, comprising the following steps:
a) providing a hydrogel which contains at least one further component which precipitates or forms a solid phase upon application of an electric field to said hydrogel,
b) applying an electric field to the hydrogel,
c) inducing a structurization, preferably a pore formation in the hydrogel.

2. The method according to claim 1, wherein steps b) and c) are performed at the same time, one before the other, or one after the other or such that one of the two steps is commenced after the respective other step has been started without the other step having yet been completed.

3. The method according to any one of the preceding claims, **characterized in that** step c) is performed by a deep-freezing process and/or by lyophilization of the hydrogel and/or by electrolysis of water and/or by electrolysis of aqueous solutions in the hydrogel.

4. The method according to any one of the preceding claims, **characterized in that** step b) is performed by means of at least two electrodes of opposite polarization.

5. The method according to any one of the preceding claims, **characterized in that** in step b), the at least one further component which precipitates or forms a solid phase upon application of an electric field to the hydrogel forms a crystalline and/or amorphous phase or a combination of crystalline and amorphous phases.

6. The method according to any one of the preceding claims, **characterized in that** in step b) a voltage of 3 V to 20 V is applied to the hydrogel and/or a current of an amperage of 0.5 A to 5 A is passed through the hydrogel, preferably over a period of 0.5 minutes to 120 minutes.

7. The method according to claim 6, **characterized in that** the applied voltage is a direct voltage or an alternating voltage.

8. The method according to any one of the preceding claims, **characterized in that** the hydrogel is a hydrogel of one or more compound(s) selected from the group comprising collagen, in particular type I and II collagen, telopeptide-free collagen, collagen hydrosylates, proteoglycanes, glycosaminoglycanes, polymethacrylic acids, polymethacrylates, polyvinyl pyrrolidone, polyvinyl alcohol, gelatin, polyglycolic acid, polylactic acid, copolymers of polylactic acid and polyglycole acid, glucose, lipids, phospholipids, urates, hyaluronioc acid, hyaluronic acid derivatives, in particular hyaluronic acid esters, and ionic constituents selected from the group comprising Na⁺, K⁺, Mg²⁺, Ca²⁺, Cl⁻, HCO₃⁻, HPO₄²⁻, SO₄²⁻, F⁻.

9. The method according to any one of the preceding claims, **characterized in that** the at least one further component which precipitates or forms a solid phase upon application of an electric field to the hydrogel is selected from the group comprising calcium carbonates, calcium phosphates, in particular hydroxylapatite, tricalcium phosphates, brushite, octacalcium phosphate, amorphous calcium phosphate, tetracalcium phosphate, monetite, calcium-deficient hydroxylapatite as well as compounds formed from ionic constituents selected from the group comprising Na⁺, K⁺, Mg²⁺, Ca²⁺, Cl⁻, HCO₃⁻, HPO₄²⁻, SO₄²⁻, F⁻.

10. The method according to any one of the preceding claims, **characterized in that** the hydrogel comprises a component which is electrically conductive, said component being identical to or different from the at least one further component which precipitates or forms a solid phase upon application of an electric field to the hydrogel.

11. The method according to claim 10, **characterized in that** the electrically conductive component is introduced into the hydrogel or applied to the hydrogel.

12. The method according to claim 10 or 11, **characterized in that** the electrically conductive component is chemically and/or biologically inert.

13. The method according to claim 12, **characterized in that** the electrically conductive component is selected from the group comprising noble metals, in particular elementary gold and/or platinum, as well as carbon, in particular graphite.

14. The method according to any one of claims 11 - 13, **characterized in that** insofar as the electrically conductive component is introduced into the hydrogel, it is homogenously or non-homogenously distributed therein.

15. The method according to any one of claims 11 - 13, **characterized in that** insofar as the electrically conductive component is applied to a surface of the hydrogel, it is structured by a surface treatment.

16. The method according to any one of the preceding claims, **characterized in that** the hydrogel is present as a layer which is wound prior to or after the executing of step b) and/or c).

17. The method according to any one of the preceding claims, **characterized in that** the hydrogel is chemically and/or physically cross-linked.

18. The method according to any one of claims 3 - 17, **characterized in that** the lyophilization is performed by deep-freezing the hydrogel to a temperature in the range of -1°C to -196°C and subsequent sublimation.

19. The method according to claim 18, **characterized in that** the lyophilization of the hydrogel is performed in a directional and/or non-directional manner, preferably from one and/or more side(s) of the hydrogel.

20. The method according to one of the claims 18 or 19, **characterized in that** the lyophilization of the hydrogel occurs over a period of approximately half an hour to four hours.

21. A composite material produced by a method according to one of the preceding claims.

22. The composite material according to claim 21, **characterized by** a pore-exhibiting layer of a gel, preferably a partially hydrated hydrogel or a xerogel, to which is linked a solid phase, preferably a crystalline and/or amorphous phase or a combination of crystalline and amorphous phases.

23. The composite material according to any one of claims 21 or 22, **characterized by** a pore size of 10 µm to 150 µm.

24. The composite material according to any one of claims 21 - 23, **characterized in that** the solid, preferably crystalline and/or amorphous phase or the combination of crystalline or amorphous phases is a calcium phosphate.

25. The composite material according to any one of claims 21 - 24, further comprising at least one substance promoting cell growth or cell colonization or cell adhesion.

26. The composite material according to claim 25, **characterized in that** the at least one substance promoting cell growth or cell colonization or cell adhesion is a growth factor or a fetal serum or poly-L-lysine, wherein the growth factor is preferably selected from the group comprising substances of the TGF-β superfamily, in particular TGF-β1, and wherein the fetal serum is an animal fetal serum, for example fetal calf serum.

27. The composite material according to claim 25, **characterized in that** the at least one substance promoting cell growth or cell colonization or cell adhesion is a serum, wherein the serum is of an autogenous, syngenous, allogenous or xenogenous origin.

28. The composite material according to any one of claims 21 - 27, **characterized in that** it further comprises biological cells, preferably human or animal cells or plant cells.

29. Use of a composite material according to any one of claims 21 - 28 as a carrier medium for biological cells, preferably human or animal cells or plant cells.

30. The composite material according to any one of claims 21 - 28 for use as tissue replacement in the human or animal body.

## Revendications

1. Procédé de fabrication d'un matériau composite apte à être utilisé comme substitut osseux / cartilagineux, comportant les étapes suivantes :
a) mise à disposition d'un hydrogel comportant au moins un autre composant qui précipite ou forme une phase solide en cas d'application d'un champ électrique à l'hydrogel ;
b) application d'un champ électrique à l'hydrogel ;
c) induction d'une structuration, de préférence d'une formation de pores dans l'hydrogel.

2. Procédé selon la revendication 1, où les étapes b) et c) sont effectuées au même moment, l'une avant l'autre ou l'une après l'autre ou de telle façon que l'une des deux étapes est commencée après le début de l'autre étape sans que l'autre étape soit déjà terminée.

3. Procédé selon l'une des revendications précédentes, **caractérisé en ce que** l'étape c) est effectuée par un processus de congélation et/ou par lyophilisation de l'hydrogel et/ou par électrolyse d'eau et/ou par électrolyse de solutions aqueuses dans l'hydrogel.

4. Procédé selon l'une des revendications précédentes, **caractérisé en ce que** l'étape b) est effectuée au moyen d'au moins deux électrodes inversement polarisées.

5. Procédé selon l'une des revendications précédentes, **caractérisé en ce qu'**à l'étape b) l'au moins un autre composant qui précipite ou forme une phase solide en cas d'application d'un champ électrique à l'hydrogel, forme une phase cristalline et/ou amorphe ou une combinaison de phases cristallines et amorphes.

6. Procédé selon l'une des revendications précédentes, **caractérisé en ce qu'**à l'étape b) une tension de 3 V à 20 V est appliquée à l'hydrogel et/ou un courant d'une intensité de 0,5 A à 5 A parcourt l'hydrogel, de préférence pendant une période de 0,5 minute à 120 minutes.

7. Procédé selon la revendication 6, **caractérisé en ce que** la tension appliquée est une tension continue ou une tension alternative.

8. Procédé selon l'une des revendications précédentes, **caractérisé en ce que** l'hydrogel est un hydrogel d'une ou de plusieurs liaison(s) sélectionnées parmi le groupe comprenant le collagène, en particulier le collagène de type I et II, le collagène exempt de télopeptide, les hydrolysats de collagène, les protéoglycanes, les glycosaminoglycanes, les acides polyméthacryliques, les polyméthacrylates, la polyvinylpyrrolidone, l'alcool de polyvinyle, la gélatine, l'acide de polyglycol, l'acide polylactique, les copolymères d'acide polylactique et d'acide de polyglycol, le glucose, les lipides, les phospholipides, les urates, l'acide hyaluronique, les dérivés d'acide hyaluronique, en particulier les esters d'acide hyaluronique, ainsi que les composants ioniques sélectionnés parmi le groupe comprenant Na⁺, K⁺, Mg²⁺, Ca²⁺, Cl⁻, HCO₃⁻, HPO₄²⁻, SO₄²⁻, F⁻.

9. Procédé selon l'une des revendications précédentes, **caractérisé en ce que** l'au moins un autre composant qui précipite ou forme une phase solide en cas d'application d'un champ électrique à l'hydrogel, est sélectionné parmi le groupe comprenant les carbonates de calcium, les phosphates de calcium, en particulier l'hydroxylapatite, les triphosphates de calcium, la brushite, l'octaphosphate de calcium, le phosphate de calcium amorphe, le tétraphosphate de calcium, la monétite, l'hydroxylapatite déficitaire en calcium, ainsi que des liaisons formées de composants ioniques sélectionnés parmi le groupe comprenant Na⁺, K⁺, Mg²⁺, Ca²⁺, Cl⁻, HCO₃⁻, HPO₄²⁻, SO₄²⁻, F⁻.

10. Procédé selon l'une des revendications précédentes, **caractérisé en ce que** l'hydrogel comporte un composant qui est électriquement conductible, ce composant étant identique à, ou différent de, l'au moins un autre composant qui précipite ou forme une phase solide en cas d'application d'un champ électrique à l'hydrogel.

11. Procédé selon la revendication 10, **caractérisé en ce que** le composant électriquement conductible est introduit dans l'hydrogel ou appliqué sur l'hydrogel.

12. Procédé selon l'une des revendications 10 ou 11, **caractérisé en ce que** le composant électriquement conductible est chimiquement et/ou biologiquement inerte.

13. Procédé selon la revendication 12, **caractérisé en ce que** le composant électriquement conductible est sélectionné parmi le groupe comprenant les métaux nobles, en particulier l'or élémentaire et/ou le platine, ainsi que le carbone, en particulier le graphite.

14. Procédé selon l'une des revendications 11 à 13, **caractérisé en ce que**, dans la mesure où le composant électriquement conductible est introduit dans l'hydrogel, il y est réparti de façon homogène ou inhomogène dans l'hydrogel.

15. Procédé selon l'une des revendications 11 à 13, **caractérisé en ce que**, dans la mesure où le composant électriquement conductible est appliqué sur une surface de l'hydrogel, il est structuré par un traitement de surface.

16. Procédé selon l'une des revendications précédentes, **caractérisé en ce que** l'hydrogel est présent comme une couche qui est roulée avant ou après l'exécution de l'étape b) et/ou c).

17. Procédé selon l'une des revendications précédentes, **caractérisé en ce que** l'hydrogel est chimiquement et/ou physiquement réticulé.

18. Procédé selon l'une des revendications 3 à 17, **caractérisé en ce que** la lyophilisation est effectuée par congélation de l'hydrogel à une température de l'ordre de -1°C à -196°C et sublimation consécutive.

19. Procédé selon la revendication 18, **caractérisé en ce que** la congélation de l'hydrogel est effectuée de façon dirigée et/ou non dirigée, de préférence à partir d'un côté et/ou de plusieurs côtés de l'hydrogel.

20. Procédé selon l'une des revendications 18 à 19, **caractérisé en ce que** la congélation de l'hydrogel est effectuée pendant une période d'approximativement une demi-heure à quatre heures.

21. Matériau composite fabriqué par un procédé selon l'une des revendications précédentes.

22. Matériau composite selon la revendication 21, **caractérisé par** une couche présentant des pores d'un gel, de préférence d'un hydrogel partiellement hydraté ou d'un xérogel, à laquelle est jointe une phase solide, de préférence une phase cristalline et/ou amorphe ou une combinaison de phases cristallines et amorphes.

23. Matériau composite selon l'une des revendications 21 à 22, **caractérisé par** une grosseur de pore de 10 µm à 150 µm.

24. Matériau composite selon l'une des revendications 21 à 23, **caractérisé en ce que** la phase solide, de préférence cristalline et/ou amorphe ou la combinaison de phases cristallines et amorphes, est un phosphate de calcium.

25. Matériau composite selon l'une des revendications 21 à 24, comprenant en outre au moins une substance favorisant la croissance ou l'implantation ou l'adhésion de cellules.

26. Matériau composite selon la revendication 25, **caractérisé en ce que** l'au moins une substance favorisant la croissance ou l'implantation ou l'adhésion de cellules est un facteur de croissance ou un sérum foetal ou une poly-L-lysine, le facteur de croissance étant de préférence sélectionné parmi le groupe comprenant les substances de la superfamille du TGF-β, en particulier du TGF-β1, et le sérum foetal étant un sérum foetal animal, par exemple un sérum foetal de veau.

27. Matériau composite selon la revendication 25, **caractérisé en ce que** l'au moins une substance favorisant la croissance ou l'implantation ou l'adhésion de cellules est du sérum, le sérum étant d'origine autogène, syngène, allogène ou xénogène.

28. Matériau composite selon l'une des revendications 21 à 27, **caractérisé en ce qu'**il comprend en outre des cellules biologiques, de préférence des cellules humaines ou animales ou des cellules végétales.

29. Utilisation d'un matériau composite selon l'une des revendications 21 à 28 comme support pour des cellules biologiques, de préférence des cellules humaines ou animales ou des cellules végétales.

30. Matériau composite selon l'une des revendications 21 à 28, destiné à être utilisé comme substitut de tissu dans le corps humain ou animal.
